# DEMANDE DE BREVET EUROPEEN

(11) **EP 1 002 527 A2**
(43) Date de publication de la demande: **24.05.2000**
(21) Numéro de dépôt: 99401956.0
(22) Date de dépôt: 30.07.1999
(51) Int. Cl.: A61K 7/48, C08L 71/02

(54) **Composition aqueuse gélifiée stable à forte teneur en électrolyte**

(30) Priorité: 25.09.1998 FR 9812041
(71) Demandeur: L'OREAL, 75008 Paris (FR)
(72) Inventeur: Chevalier, Véronique, 94440 Villecresnes (FR)
(74) Mandataire: Rasson, Catherine

(57) **Abrégé**

La présente invention se rapporte à une composition topique, caractérisée en ce qu'elle comprend dans un milieu topiquement acceptable (1) de l'eau, (2) au moins un électrolyte et/ou au moins un alcool primaire, (3) au moins un polyuréthanne associatif et (4) au moins un polymère oxyalkyléné choisi parmi les polyéthylène glycols, les polypropylène glycols, les dérivés d'acides gras et de polyéthylène glycol et/ou de polypropylène glycol, les dérivés glycérylés d'acides gras et de polyéthylène glycol et/ou de polypropylène glycol.

La composition obtenue reste stable même en présence d'une quantité importante d'électrolyte ou d'alcool primaire, et elle peut constituer un gel ou une émulsion.

La composition de l'invention peut être notamment utilisée dans les domaines cosmétique et/ou dermatologique pour le traitement, la protection et/ou le soin de la peau, du cuir chevelu, des muqueuses, des ongles et/ou des cheveux.

## Description

La présente invention se rapporte à une composition gélifiée stable, notamment topique, pouvant contenir une quantité importante d'électrolyte ou d'alcool primaire, et à son utilisation notamment pour le traitement, la protection et/ou le soin de la peau, du cuir chevelu, des muqueuses, des ongles et/ou des cheveux.

Dans les domaines cosmétique, dermatologique et pharmaceutique, il est connu d'utiliser des compositions topiques sous forme de gels ou d'émulsions comportant des gélifiants qui donnent de la consistance à ces compositions. La majorité des gélifiants utilisés classiquement sont des gélifiants aqueux et notamment des polymères carboxyvinyliques, que l'on neutralise par une base.

Il arrive cependant que certains composés que l'on souhaite utiliser dans ces compositions ne permettent pas l'emploi des gélifiants cités précédemment par suite d'incompatibilité.

On sait par exemple que les électrolytes (sels inorganiques et organiques) "cassent" les émulsions gélifiées par les polymères carboxyvinyliques et les liquéfient. Ainsi, des compositions contenant des polymères carboxyvinyliques et des électrolytes sont dépourvues de consistance, ce qui va à l'encontre du résultat recherché par l'emploi d'un gélifiant.

Or, il peut être souhaitable d'introduire des électrolytes dans des compositions épaissies, notamment topiques, et parfois même en une quantité relativement importante, notamment quand ces électrolytes ont un effet bénéfique sur la peau ou les cheveux.

Une solution consiste à utiliser, à la place des polymères carboxyvinyliques, des gélifiants de type polysaccharidique tels que les gommes guar ou xanthane ou les dérivés cellulosiques. Ainsi, le document EP-A-654270 décrit une composition topique destinée au traitement de l'acné et des dermatites séborrhéiques, comportant un mélange de sels et, comme gélifiant, un dérivé de cellulose tel que l'hydroxyéthylcellulose.

Malheureusement, les compositions à base de dérivés de cellulose conformes à ce document, et notamment les gels aqueux ne comportant pas de phase grasse, n'ont pas une texture lisse et présentent au contraire un aspect grumeleux peu agréable à voir. En outre, ils laissent la peau « comme mouillée » après application, ces compositions ne pénétrant pas suffisamment dans la peau. Tout ceci rend leur utilisation rédhibitoire dans les domaines cosmétique et/ou dermatologique.

L'association de ces dérivés de cellulose avec un autre agent épaississant tel qu'un silicate, comme décrit par exemple dans le document WO-A-93/8230, donne des compositions où subsistent les mêmes inconvénients qu'indiqués ci-dessus (aspect grumeleux).

Par ailleurs, l'incorporation d'alcool primaire et notamment d'éthanol dans les compositions gélifiées pose les mêmes problèmes de stabilité. Or, il est important de pouvoir utiliser des compositions gélifiées contenant un alcool primaire car la solubilisation d'actifs rend souvent nécessaire l'utilisation d'alcool primaire comme solvant.

Il subsiste donc le besoin de compositions contenant des électrolytes et/ou un alcool primaire et ne présentant pas les inconvénients rencontrés avec les gélifiants connus, notamment d'inconsistance, d'instabilité, d'aspect grumeleux, de sensation désagréable au toucher et/ou d'incompatibilité avec les électrolytes et/ou les alcools primaires.

La demanderesse a trouvé de façon inattendue une classe de polymères associatifs permettant, en association avec un polymère oxyalkyléné, de stabiliser les compositions contenant une forte teneur d'électrolyte(s) et/ou d'alcool(s) primaire(s).

Aussi, la présente invention a pour objet une composition topique, caractérisée en ce qu'elle comprend dans un milieu topiquement acceptable, (1) de l'eau, (2) au moins un électrolyte et/ou au moins un alcool primaire, (3) au moins un polyuréthanne associatif et (4) au moins un polymère oxyalkyléné choisi parmi les polyéthylène glycols, les polypropylène glycols, les dérivés d'acides gras et de polyéthylène glycol et/ou de polypropylène glycol, les dérivés glycérylés d'acides gras et de polyéthylène glycol et/ou de polypropylène glycol.

La composition selon la présente invention a une viscosité allant de préférence de 0,1 à 50 Pa.s (1 à 500 poises) et plus particulièrement de 0,5 à 10 Pa.s (5 à 100 poises), ladite viscosité étant mesurée au viscosimètre Rhéomat 180 à 25°C et à un taux de cisaillement de 200 s⁻¹. Ce peut être aussi bien un gel aqueux qu'une émulsion ou dispersion.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes alkyle ayant de 6 à 30 atomes de carbone, séparées par une séquence hydrophile, les chaînes alkyle pouvant être des chaînes pendantes ou des chaînes en bout de séquence hydrophile. En particulier, il est possible qu'une ou plusieurs chaînes pendantes soient prévues. En outre, le polymère peut comporter une chaîne alkyle à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, ces polymères sont des copolymères triblocs dont la séquence hydrophile est une chaîne polyoxyéthylénée comportant de 50 à 1000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquences hydrophiles sont liées par d'autres liaisons chimiques aux séquences lipophiles.

A titre d'exemple des polymères associatifs utilisables dans l'invention, on peut citer le polymère C₁₆-OE₁₂₀-C₁₆ vendu par la société HÜLS sous le nom Sérad FX1100 (molécule à fonction uréthanne et poids moléculaire moyen de 1300, OE étant un motif oxyéthyléné). Comme polymère associatif, on peut aussi utiliser le Rhéolate 205 à fonction urée vendu par la société RHEOX ou encore le Rhéolate 208 ou 204. Ces polyuréthannes associatifs sont vendus sous forme pure.

On peut aussi utiliser des solutions ou dispersions de ces polymères notamment dans l'eau ou en milieu hydroalcoolique. A titre d'exemple de tels polymères on peut citer le Sérad FX1010 et le Sérad 1035 vendus par la société HÜLS, le Rhéolate 255, le Rhéolate 278 et le Rhéolate 244 vendus par la société RHEOX. On peut aussi utiliser le produit DW 1206F et le DW 1206J, ainsi que l'Acrysol RM 184, l'Acrysol 44 ou l'Acrysol 46 vendus par la société ROHM & HAAS. On peut aussi utiliser le produit DW 1206B vendu par la société ROHM & HAAS comportant une chaîne alkyle en C₂₀ et une liaison uréthanne, vendu à 20 % en matière sèche dans l'eau.

Selon un mode préféré de réalisation de l'invention, on utilise la solution de « Acrysol 46 » à 15 % en matière active.

La composition selon l'invention contient un ou plusieurs polyuréthannes associatifs en une quantité suffisante pour assurer le résultat escompté, c'est-à-dire la stabilité de la composition gélifiée, cette quantité allant par exemple de 0,1 à 40 % et de préférence de 0,5 à 20 % en poids de matière active par rapport au poids total de la composition.

Dans la présente invention, le polymère est choisi parmi les polyéthylène glycols, les polypropylène glycols, les dérivés d'acides gras et de polyéthylène glycol et/ou de polypropylène glycol, les dérivés glycérylés d'acides gras et de polyéthylène glycol et/ou de polypropylène glycol.

Selon un mode préféré de réalisation de l'invention, le polymère oxyalkyléné comporte de 2 à 100 et de préférence de 4 à 80 groupes oxyéthylénés et/ou oxypropylénés.

Comme polymère oxyalkyléné utilisable dans la composition de l'invention, on peut citer par exemple, parmi les polyéthylène glycols et polypropylène glycols, le PEG-20, le PEG-32, le PPG-15; parmi les dérivés d'acides gras et de polyéthylène glycol et/ou de polypropylène glycol, notamment les stéarates, oléates et laurates de polyéthylène glycol et/ou de polypropylène glycol, plus particulièrement les stéarates tels que le stéarate de polyéthylène glycol à 50 groupes oxyéthylénés (PEG-50 stearate en nom CTFA) et l'éther de polypropylène glycol d'alcool stéarylique à 15 groupes oxypropylénés (PPG-15 stearyl ether en nom CTFA). On peut citer parmi les dérivés glycérylés d'acides gras et de polyéthylène glycol ou de polypropylène glycol, le stéarate de glycéryle et de PEG-30. On entend ci-dessus par acide gras les acides ayant une chaîne alkyle comportant de 8 à 30 atomes de carbone.

On peut utiliser un ou plusieurs de ces polymères oxyalkylénés.

La quantité de polymère oxyalkyléné peut varier dans une large mesure. Elle peut aller par exemple de 0,1 à 10 % et de préférence de 1 à 5 % en poids par rapport au poids total de la composition.

On entend par alcool primaire les alcools qui comportent un seul groupement hydroxyle. Comme alcool primaire, on peut utiliser dans la composition de l'invention tout alcool aliphatique à chaîne linéaire ou ramifiée, comportant de 1 à 6 atomes de carbone et de préférence de 2 à 4 atomes de carbone. Comme alcool primaire, on peut citer par exemple l'éthanol, le propanol et l'isopropanol. On utilise de préférence l'éthanol ou un mélange d'éthanol et d'un autre alcool primaire tel que l'isopropanol.

La quantité d'alcool(s) primaire(s) dépend du but escompté pour la composition selon l'invention et notamment de la quantité d'actif(s) à solubiliser si l'alcool est par exemple utilisé pour solubiliser un ou des actifs. L'alcool ou les alcools primaires peuvent être présents dans la composition de l'invention par exemple en une quantité allant de 1 à 20 % et de préférence de 1 à 15 % en poids par rapport au poids total de la composition.

Comme électrolyte utilisable dans la composition selon l'invention, on peut citer notamment les sels des métaux mono-, di- ou trivalents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

Le sel peut être choisi en particulier parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges.

De préférence, le sel est choisi parmi les sels de calcium, de magnésium, de sodium, de potassium et leurs mélanges, et plus particulièrement le chlorure de magnésium, le chlorure de potassium, le chlorure de sodium, le chlorure de calcium, le bromure de magnésium et leurs mélanges. Il peut s'agir en particulier d'un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange aqueux étant ici appelé "mélange des sels de la mer morte" car il correspond aux principaux sels contenus dans la mer morte.

On peut aussi utiliser ces sels sous forme d'une solution ou d'une eau les contenant, et notamment sous forme d'une eau thermale ou minérale. En général, une eau minérale est propre à la consommation, ce qui n'est pas toujours le cas d'une eau thermale. Chacune de ces eaux contient, entre autre, des minéraux solubilisés et des oligoéléments.

L'eau thermale ou l'eau minérale utilisée peut être choisie par exemple parmi l'eau de Vittel, les eaux du bassin de Vichy, l'eau d'Uriage, l'eau de La Roche Posay, l'eau de La Bourboule, l'eau d'Enghien-les-Bains, l'eau de Saint-Gervais-les Bains, l'eau de Néris-les-Bains, l'eau d'Allevard-les-Bains, l'eau de Digne, l'eau de Maizières, l'eau de Neyrac-les-bains, l'eau de Lons-le-Saunier, l'eau des Eaux Bonnes, l'eau de Rochefort, l'eau de Saint Christau, l'eau des Fumades, l'eau d'Avène et l'eau de Tercis-les-bains.

La quantité d'électrolyte(s) dans la composition de l'invention peut varier dans une large mesure selon le but recherché. Cette quantité peut aller par exemple de 0,5 à 40 % et de préférence de 5 à 20 % en poids par rapport au poids total de la composition.

La composition topique selon l'invention s'applique dans tous les domaines où l'on souhaite obtenir une composition épaissie en présence d'électrolytes et/ou d'alcools et notamment dans les domaines cosmétique, dermatologique et pharmaceutique.

De préférence, la composition selon l'invention est destinée à un soin, à une protection ou à un traitement topique. Dans ce cas, la composition doit contenir un milieu topiquement acceptable, c'est-à-dire compatible avec la peau, les muqueuses, les ongles, le cuir chevelu et/ou les cheveux. Elle peut se présenter sous toutes les formes galéniques appropriées pour une application topique et notamment sous forme d'émulsion ou de dispersion eau-dans-huile (E/H) ou huile-dans-eau (H/E) ou triple (E/H/E ou H/E/H). La composition selon l'invention peut aussi contenir des vésicules lipidiques ioniques et/ou non-ioniques. Elle peut constituer par exemple une crème ou une pommade.

Les quantités des différents constituants de la composition selon l'invention sont celles classiquement utilisées dans les domaines considérés.

Lorsque la composition de l'invention est une émulsion ou une dispersion, la proportion de la phase grasse peut aller de 5 à 80 % et de préférence de 5 à 50 % en poids par rapport au poids total de la composition. Les huiles, les émulsionnants et éventuellement les coémulsionnants utilisés dans la composition sous forme d'émulsion sont choisis parmi ceux classiquement utilisés dans le domaine considéré. L'émulsionnant et éventuellement le coémulsionnant sont généralement présents, dans la composition, en une proportion allant de 0,3 à 30 % et de préférence de 0,5 à 20 % en poids par rapport au poids total de la composition.

Selon un mode particulier de réalisation de l'invention, les dérivés de polymères oxyalkylénés décrits ci-dessus peuvent jouer le rôle d'émulsionnants. On peut utiliser en particulier les esters d'acide gras et de polyéthylène glycol et/ou polypropylène glycol. Comme autre émulsionnant, on peut citer par exemple les esters d'acide gras et de glycérol tels que le stéarate de glycéryle.

Comme huiles utilisables dans l'invention, on peut citer par exemple les huiles d'origine végétale (huile d'arachide), les huiles d'origine animale, les huiles de synthèse et notamment les esters gras (myristate d'isopropyle), et les mélanges de ces huiles, ainsi que les mélanges de ces huiles avec les huiles de silicone, les huiles fluorées et/ou les huiles minérales. On peut aussi utiliser comme matières grasses des alcools gras (alcool cétylique), des acides gras, des cires et composés cireux.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les filtres solaires, les bactéricides, les absorbeurs d'odeur et les matières colorantes. Les quantités de ces différents adjuvants sont celles classiquement utilisées dans le domaine considéré, et par exemple de 0,01 à 20 % en poids par rapport au poids total de la composition. Ces adjuvants, selon leur nature, peuvent être introduits dans la phase grasse, dans la phase aqueuse et/ou dans les vésicules lipidiques. Bien entendu, l'homme de l'art veillera à choisir ce ou ces éventuels adjuvants et/ou leurs quantités de manière telle que les propriétés avantageuses attachées intrinsèquement à la composition conforme à l'invention ne soient pas, ou substantiellement pas, altérées par la ou les adjonctions envisagées.

Comme actifs utilisables dans l'invention, on peut citer par exemple les hydratants tels que les polyols et notamment la glycérine, les actifs pour traiter les signes du vieillissement, l'acné, les dermatites, les taches pigmentaires, comme par exemple les vitamines telles que la vitamine C (acide ascorbique), la vitamine E (tocophérol), la vitamine A (rétinol) et leurs dérivés, en particulier leurs esters ; les bêta-hydroxyacides tels que l'acide salicylique et ses dérivés comme l'acide n-octanoyl-5-salicylique ; les alpha-hydroxyacides tels que l'acide lactique et l'acide glycolique ; l'acide rétinoique et ses dérivés ; les actifs blanchissants comme l'acide kojique et l'acide caféique.

La composition selon l'invention peut notamment être utilisée pour le traitement, la protection et/ou le soin de la peau, des muqueuses, du cuir chevelu, des ongles et/ou des cheveux, en particulier pour le traitement de la peau sensible et du cuir chevelu sensible et/ou pour hydrater la peau.

Aussi, la présente invention a encore pour objet l'utilisation cosmétique de la composition telle que définie ci-dessus pour le traitement, la protection et/ou le soin de la peau, des muqueuses, du cuir chevelu, des ongles et/ou des cheveux.

La présente invention a aussi pour objet l'utilisation de la composition telle que définie ci-dessus pour la fabrication d'une composition destinée au traitement et/ou au soin de la peau, des muqueuses, du cuir chevelu, des ongles et/ou des cheveux.

La présente invention a aussi pour objet un procédé de traitement cosmétique et/ou dermatologique de la peau, du cuir chevelu, des cheveux, des ongles et/ou des muqueuses, caractérisé par le fait que l'on applique sur la peau, le cuir chevelu, les cheveux, les ongles et/ou les muqueuses, une composition telle que définie ci-dessus.

Les exemples ci-après de composition selon l'invention sont donnés à titre d'illustration et sans caractère limitatif. Les quantités y sont données en % en poids.

### Exemple 1: Emulsion huile-dans-eau

### Phase huileuse:

- Alcool cétylique 5 %
- Stéarate de glycéryle 2 %
- Stéarate de PEG-50 2 %
- Huile d'arachide 6,2 %
- Myristate d'isopropyle 3 %

### Phase aqueuse :

- Chlorure de strontium 7 %
- Acrysol 46 (à 15 % en matière active) 6,7 %
   (soit 1 % de matière active)
- Eau déminéralisée qsp 100 %

La crème obtenue reste stable même après deux mois à 45°C. Elle peut être notamment utilisée comme crème de nuit.

### Exemple 2 : Emulsion huile-dans-eau

### Phase huileuse :

- Alcool cétylique 5 %
- Stéarate de glycéryle 2 %
- PEG-50 stearate 2 %
- Huile d'arachide 6,2 %
- Myristate d'isopropyle 3 %

### Phase aqueuse :

- Ethanol 7 %
- Acide n-octanoyl-5-salicylique 0,5 %
- Acrysol 46 (à 15 % en matière active) 6,7 %
   (soit 1 % de matière active)
- Eau déminéralisée qsp 100 %

La crème obtenue reste stable même après deux mois à 45°C. Elle peut être notamment utilisée comme crème de jour.

### Exemple comparatif 1 :

On prépare une émulsion identique à celle de l'exemple 1 ou 2 mais ne comportant pas de polymère Acrysol 46. L'émulsion obtenue ne reste pas stable après 2 mois à 45°C et se sépare en deux phases.

### Exemple comparatif 2 :

On prépare une émulsion identique à celle de l'exemple 1 ou 2 mais ne comportant pas de stéarate de PEG-50. L'émulsion obtenue n'est pas épaissie et casse rapidement en se séparant en deux phases.

### Exemple 3 : Emulsion huile-dans-eau

### Phase huileuse :

- Alcool cétylique 5 %
- Stéarate de glycéryle 2 %
- PEG-50 stearate 2 %
- Huile d'arachide 6,2 %
- Myristate d'isopropyle 3 %

### Phase aqueuse :

- Ethanol 7 %
- Acrysol 46 (à 15 % en matière active) 6,7 %
   (soit 1 % de matière active)
- Mélange de sels de la mer morte 5 %
- Eau déminéralisée qsp 100 %

La crème obtenue reste stable même après deux mois à 45°C. Elle peut être notamment utilisée comme crème de jour.

### Exemple 4 : Emulsion huile-dans-eau

### Phase huileuse :

- Alcool cétylique 5 %
- Stéarate de glycéryle 2 %
- PPG-15 stearyl ether 2 %
- Huile d'arachide 6,2 %
- Myristate d'isopropyle 3 %

### Phase aqueuse :

- Ethanol 7 %
- Sels de la mer morte 5 %
- Acrysol 46 (à 15 % en matière active) 6,7 %
   (soit 1 % de matière active)
- Eau déminéralisée qsp 100 %

La crème obtenue reste stable même après deux mois à 45°C. Elle peut être notamment utilisée comme crème de jour.

## Revendications

1. Composition topique, caractérisée en ce qu'elle comprend dans un milieu topiquement acceptable, (1) de l'eau, (2) au moins un électrolyte et/ou au moins un alcool primaire, (3) au moins un polyuréthanne associatif et (4) au moins un polymère oxyalkyléné choisi parmi les polyéthylène glycols, les polypropylène glycols, les dérivés d'acides gras et de polyéthylène glycol et/ou de polypropylène glycol, les dérivés glycérylés d'acides gras et de polyéthylène glycol et/ou de polypropylène glycol.

2. Composition selon la revendication 1, caractérisée en ce que le polyuréthanne associatif est un polymère séquencé ou greffé comportant au moins deux chaînes alkyle ayant de 6 à 30 d'atomes de carbone, séparées par une séquence hydrophile.

3. Composition selon la revendication 1 ou 2, caractérisée en ce que le polyuréthanne associatif comporte une séquence hydrophile polyoxyéthylénée.

4. Composition selon l'une quelconque des revendications précédentes, caractérisée en ce que le polyuréthanne associatif est un polymère tribloc.

5. Composition selon l'une quelconque des revendications précédentes, caractérisée en ce que la quantité de polyuréthanne(s) associatif(s) va de 0,1 à 40 % en poids de matière active par rapport au poids total de la composition.

6. Composition selon l'une quelconque des revendications précédentes, caractérisée en ce que l'alcool primaire est un alcool aliphatique à chaîne linéaire ou ramifiée comportant de 2 à 4 atomes de carbone.

7. Composition selon l'une quelconque des revendications précédentes, caractérisée en ce que l'alcool primaire est l'éthanol ou un mélange d'éthanol et d'un autre alcool primaire.

8. Composition selon l'une quelconque des revendications précédentes, caractérisée en ce que la quantité d'alcool(s) primaire(s) va de 0,1 à 20 % en poids par rapport au poids total de la composition.

9. Composition selon l'une quelconque des revendications précédentes, caractérisée en ce que la quantité d'électrolyte(s) va de 0,5 à 40 % en poids par rapport au poids total de la composition.

10. Composition selon l'une quelconque des revendications précédentes, caractérisée en ce que l'électrolyte est un sel de métal mono-, di- ou trivalent.

11. Composition selon la revendication précédente, caractérisée en ce que le sel est choisi parmi les sels de baryum, de calcium, de strontium, de sodium, de potassium, de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

12. Composition selon l'une quelconque des revendications précédentes, caractérisée en ce que l'électrolyte est constitué d'ions choisis parmi les ions chlorure, borate, bicarbonate, carbonate, nitrate, hydroxyde, sulfate, persulfate, glycérophosphate, acétate, d'α-hydroxyacides, d'acides de fruit, d'acides aminés.

13. Composition selon l'une quelconque des revendications précédentes, caractérisée en ce que l'électrolyte est choisi parmi le chlorure de magnésium, le chlorure de potassium, le chlorure de sodium, le chlorure de calcium, le bromure de magnésium et leurs mélanges.

14. Composition selon l'une quelconque des revendications précédentes, caractérisée en ce que l'électrolyte est introduit sous forme d'une eau thermale et/ou minérale.

15. Composition selon l'une quelconque des revendications précédentes, caractérisée en ce que le polymère oxyalkyléné est choisi parmi les polymères comportant de 2 à 100 groupes oxyéthylénés et/ou oxypropylénés, et leurs dérivés.

16. Composition selon l'une quelconque des revendications précédentes, caractérisée en ce que la quantité de polymère(s) oxyalkyléné(s) va de 0,1 à 10 % en poids par rapport au poids total de la composition.

17. Composition selon l'une quelconque des revendications précédentes, caractérisée en ce qu'elle a une viscosité allant de 0,1 à 50 Pa.s.

18. Composition selon l'une quelconque des revendications précédentes, caractérisée en ce qu'elle est une émulsion.

19. Composition selon la revendication précédente, caractérisée en ce qu'elle contient un actif cosmétique et/ou dermatologique.

20. Utilisation cosmétique de la composition selon l'une quelconque des revendications précédentes pour le traitement, la protection et/ou le soin de la peau, des muqueuses, du cuir chevelu, des ongles et/ou des cheveux.

21. Utilisation de la composition selon l'une quelconque des revendications 1 à 19 pour la fabrication d'une composition destinée au traitement et/ou au soin de la peau, des muqueuses, du cuir chevelu, des ongles et/ou des cheveux.

22. Procédé de traitement cosmétique de la peau, du cuir chevelu, des cheveux, des ongles et/ou des muqueuses, caractérisé par le fait que l'on applique sur la peau, le cuir chevelu, les cheveux, les ongles et/ou les muqueuses, une composition selon l'une quelconque des revendications 1 à 19.
